# EUROPEAN PATENT APPLICATION

(11) **EP 4 632 753 A1**
(43) Date of publication of application: **15.10.2025**
(21) Application number: 24382371.3
(22) Date of filing: 10.04.2024
(51) Int. Cl.: G16H 20/17

(54) **MEDICAL SYSTEM FOR DOCUMENTING AND MANAGING A DRUG ADMINISTRATION PROCEDURE, METHOD OF DOCUMENTING AND MANAGING A DRUG ADMINISTRATION PROCEDURE, AND COMPUTER PROGRAM**

(71) Applicant: B. Braun Melsungen AG, 34212 Melsungen (DE)
(72) Inventor: PLA, Marcel, 08100 Mollet (ES); ESPAÑOL, Gerard, 08022 Barcelona (ES)
(74) Representative: Winter, Brandl - Partnerschaft mbB

(57) **Abstract**

The disclosure relates to a medical system (1) for documenting and managing a drug administration procedure, comprising: a server (2), at least one infusion pump (4) configured to administer an infusion to a patient and comprising status information (14), the at least one infusion pump (4) being in data communication with the server (2) and providing the status information (14) to the server (2), and a mobile device (8) configured to receive input information (15) from a user, the mobile device (8) being in data communication with the server (2) and providing the input information (15) to the server (2). The server (2) is configured to determine whether a protocol of documentation of the administration procedure is followed and to submit a notification (16) comprising an instruction and/or a warning to the mobile device (8) based on the status information and on the input information. The disclosure further relates to a method of documenting and managing a drug administration procedure as well as a computer program.

## Description

### Technical field

The present disclosure relates to a medical system for documenting and managing a medical drug administration procedure, particularly a clinical trial. The present disclosure also relates to a drug administration managing and documentation method and a computer program comprising instructions which, when executed by a computer, cause the computer to perform the drug administration managing and documentation method.

### Background of the disclosure

Conventionally, medical drug administration procedures with complex regimen of medication are critical and challenging and demand additional effort to ensure the accurate compliance and documentation. This can be ensured with administration protocols. An administration protocol is a document that is used to define and manage the drug administration procedure and comprises an administration regimen as well as treatment parameters, e.g. temperature, electrocardiogram, vital parameters that have to be taken at defined points in time throughout the administration procedure. The administration regimen defines specifics about the administration of drugs, such as the drug name, the flow rate, or the duration of the administration. Therefore, the administration protocol includes in detail when and how these treatment parameters should be registered depending on the different drugs to be administered.

Even more critical are clinical trials in which non-compliance with a protocol and/or regimen can lead to the invalidity of the clinical trial. A clinical trial is a form of a clinical research and study in which a medical intervention is tested on human participants. It is a primary means of determining if a new treatment or prevention strategy, such as a new drug or drug dose is safe and effective for use in patients. Conventionally, clinical trials generate data on dosage, safety and effectiveness and are designed to learn/ find out if a new treatment is more effective or has less harmful side effects than existing ones. Clinical trials, particularly in the field of oncology, require a rigid and strictly defined (clinical trial) documentation protocol that needs to be followed. Therefore, the clinical trial protocol includes in detail when and how these clinical trial parameters should be registered depending on the different drugs to be administered.

### Related Art

Numerous software solutions that manage and facilitate drug administration procedures, and in particular clinical trials, exist in the related art. For example, technologies that improve the feasibility and patient matching operations are known that are able to reduce a manual burden of matching patients to clinical trials. However, said solutions do not provide exact information/ instructions for nurses or operators to follow, in order to make sure that treatment parameters, and in particular clinical trial parameters, are documented at the right time. Conventionally, the documentation of parameters during an administration procedure is carried out or noted down by utilizing pen and paper, thus, an additional step of manually transcribing the documented parameters is necessary.

Particularly, in the case of clinical trials, if during a clinical trial the administration regimen of the clinical trial protocol has not been followed correctly and the measurements or documentations that define clinical trial parameters have been taken at the wrong time during the clinical trial or have not been taken at all, the entire clinical trial might lose its validity. This leads to the necessity to repeat clinical trials which usually causes additional work and, therefore, results in higher procedure costs.

Therefore, it is an object of the present disclosure to avoid or at least mitigate the disadvantages described above and, in particular, to provide a medical system for documenting and managing medical drug administration procedures, in particular clinical trials, that optimizes and ensures an organized administration procedure with a transparent treatment parameter documentation process, resulting in a risk reduction of documenting treatment parameters at the wrong time and therefore, performing inaccurate administration procedures (or clinical trials) and, in the worst case, in invalid administration procedures that need to be repeated. Furthermore, a method of documenting and managing administration procedures that facilitates a safe and accurate documentation of administration procedures, as well as a computer program comprising instructions which, when executed by a computer, cause the computer to perform the method of documenting and managing administration procedures should be provided.

### Brief description of the disclosure

The present disclosure provides a medical system according to claim 1, a method of documenting and managing a drug administration procedure according to claim 13 und a computer program according to claim 15. Further advantageous developments and embodiments of the disclosure are subject-matter of the dependent claims and/or are described herein below.

The present disclosure, therefore, relates to a medical system for documenting and managing a drug administration procedure/ a medical treatment procedure, in particular a clinical trial, comprising a server/ a control unit; at least one infusion pump configured to administer an infusion to a patient and comprising (infusion pump) status information, the at least one infusion pump being in data communication with the server and providing the status information (preferably directly/ in real time) to the server; and a mobile device, in particular a personal digital assistant, configured to receive input information from a user, e.g. a nurse/ operator, the mobile device being in data communication with the server and providing/ transferring the input information (preferably directly/ in real time) to the server. The server is configured to determine whether a protocol of documentation of the administration procedure or, in particular, a (clinical) trial protocol is followed and to submit (preferably directly/ in real time) a notification comprising an instruction and/or a warning/ notice to the mobile device based on the status information and on the input information. The medical system according to the present disclosure thus provides safety to the user in the process of administering the infusion (e.g. comprising a specific medication) and of registering the input information, e.g. parameters. Preferably, everything is done at the scheduled time and errors as well as misinterpretations are avoided.

In other words, the server of the medical system has access to the protocol of documentation of the administration procedure, which contains administration and documentation guidelines for the administration procedure. The server is configured to receive status information of the at least one infusion pump during the administration procedure. Furthermore, since the mobile device, which may be a personal digital assistant (PDA), hand held, tablet, notebook, a computer, or any device on which parameters can be recorded and exchanged with the server, provides the input information to the server and receives data/ information from the server that are output by the mobile device as a notification/ indication for the user, a two-way communication between the mobile device and the server is established. Since the server preferably controls the infusion/ drug administration of the infusion pump and receives - according to the present disclosure - status information of the infusion pump, in an advantageous manner also a two-way communication between the server and the infusion pump is provided. In particular, real time (two-way) communications between the server and the at least one infusion pump as well as between the server and the mobile device are established. The information that needs to be indicated by the mobile device depends on the determination result of the server, in particular on whether the protocol of documentation is being complied with and can be indicated in the form of notices/ warnings and/or instructions for the user. More specifically, the instructions comprise information about when and how the user should provide the input information to the mobile device depending on the administration protocol, in particular the clinical trial protocol. Furthermore, the mobile device is preferably configured to show step-by-step instructions to the user, in particular including data fields to insert (measured) treatment parameters. Said instructions are based on the protocol of documentation of the administration procedure, ensuring that the protocol is followed by the user who submits the input information.

The input information comprises data about whether individual parameters have been registered/ submitted or not (by the user), and/or values of the (treatment) parameters that are measured and entered into the mobile device. Examples for parameters that can be registered by the mobile device are electrocardiograms, temperature, or vital parameters. Preferably, the server can be configured to record and store the input information provided by the mobile device as well as store every action carried out on the mobile device by the user, so that the recorded data can be searchable in an audit report at a later time.

In yet another words, the mobile device can be used to register treatment parameters as input information, to cancel aspects of the administration procedure, add medication/ drugs to the administration procedure, and/or to register a change of location of the patient. Additionally or alternatively, the mobile device can be in an "empty" state when it is not connected to the medical system, meaning the mobile device does not save critical information related to the administration/ treatment procedure or patient, etc. in this state.

By issuing (real-time) notices, warnings or instructions to the user during the administration procedure, a deviation/ discrepancy from the protocol of documentation can be prevented. Therefore, the process of the administration procedure is clearly shown to the user/ operator and a possible omission of important parameters that need to be input by the user via the mobile device can be avoided more reliably. Hence, more accurate and organized administration procedures can be enabled by the disclosed medical system. E.g. in case the administration procedure is a clinical trial process there may be provided a special section for clinical trial alerts (on the mobile device), where the user receives the notice or notification when there is a procedure of/ from the clinical trial that must be registered. The notice or notification may be linked with a time point and with an administration status, which is preferably based on information received from the infusion pump.

In a further advantageous embodiment of the disclosure, the server of the medical system can be configured to store drug administration information/ administration regimen and/or to receive the drug administration information from a hospital information system (HIS) that is in data communication with the server.

Expressed differently, the server is able to access drug administration information comprising information about the set of drugs to be administered to the patient following a specific order of administration/ a detailed systematic plan, that is either stored/ saved on the server itself or delivered by a hospital information system. For example, a hospital information system can be a pharmacy management system, an oncology management system, an enterprise resource planning (ERP), a patient data management system (PDMS), or any other source from which an administration regimen can be received. The administration regimen may also vary depending on the current cycle and day of the administration. Preferably, the hospital information system is able to receive data about the administration in the form of recorded data by the server. Alternatively, the hospital information system can be replaced by another data source for providing administration information. For instance, information on the administration regimen could also be provided manually through manual user inputs. Additionally, the protocol of documentation of the administration procedure of the disclosed system is a combination of the drug administration information and the input information, preferably in the form of treatment parameters that are, preferably measured and, submitted by the user via the mobile device. Some parameters (i.e. patient data) can also be introduced directly via a web application. The protocol of documentation comprises detailed information about when and how the input information should be registered depending on the different drugs to be administered. In particular, the boundaries or conditions for the protocol of documentation can be set by a promoter that, conventionally is a company responsible for the administration procedure management. Particularly, the promoter can give the specific instructions to the hospital regarding the administration requirements and treatment parameters to be registered as input information.

Further preferably, the server can be configured to send infusion order information based on the drug administration information to the at least one infusion pump. Therefore, the server and the at least one infusion pump can be in a two-way data communication with one communication path being the status information that is sent by the at least one infusion pump to the server. In particular, the infusion order information is based on the protocol of documentation of the administration procedure. In other words, the infusion order information sent by the server follows the protocol that is also dependent upon the administration regimen/ the different kinds of drugs.

In yet another further advantageous embodiment of the disclosure, the server can be configured to receive an approval input that approves the protocol of documentation of the administration procedure and/or any changes made to the protocol of documentation before sending the infusion order information based on the drug administration information to the at least one infusion pump.

More specifically and in other words, the protocol of documentation of the administration procedure can be configured from a Back Office and the server of the disclosed medical system can be configured to be accessible through two different user profiles, an administration procedure coordinator profile and an administration procedure approver profile. Depending on the profile a protocol can have three possible statuses, namely "draft", "under review" and "approved". The administration procedure coordinator profile creates the protocol of documentation of the administration procedure by setting up the input information with respect to the drug administration information based on the promoter's instructions. These configurations are independent for each cycle and day of the treatment. In order for the protocol to be effective, a final approval is required from the administration procedure approver profile. Preferably, any change done in the protocol needs to be approved by the approver profile again. In other words, any modifications of the protocol done by the administration procedure coordinator profile will also modify the status of the protocol to "under review" and will need to be approved again by the administration procedure approver profile. Preferably, any change of the status of the protocol will also inform the medical staff involved, e.g. via email. This prevents the protocol of documentation to be modified unnecessarily and also provides another safety layer to the medical system.

In another further preferred embodiment of the disclosure, the status information provided by the at least one infusion pump and/ or the input information provided by the mobile device can comprise time stamp information. In other words, the server of the medical system that is receiving the status information from the at least one infusion pump as well as the input information from the mobile device, that is particularly submitted/ registered by the user, can also receive information about at what exact time the (infusion pump) status information and the input information have been registered/ measured/ recorded by the at least one infusion pump or the mobile device, respectively. The information (status information and/or input information) may be displayed in real time on the mobile device. The information may be stored e.g. in a storage so that the user can review the information later. Providing and preferably storing real time information of what is happening in a treatment at preferably all times makes it possible to continuously observe the information, e.g. in a web dashboard or in a PDA.

Further preferably, the server can be configured to determine if the input information is being received in an established order and/or in a timely manner and/or if any of the input information has been omitted, based on the time stamp information, and to submit a warning to the mobile device if the input information has not been received in the established order or in a timely manner. In other words, all the (drug) administration steps should preferably be registered in an established order and the medical system will alert of any omission in relation to the drug administration or any parameter registration/ input information. Preferably, the server can also be configured to analyze/ compare the status information, preferably including the time stamp information, provided by the at least one infusion pump and/ with the input information, preferably including the time stamp information, received by the mobile device to determine if the protocol of documentation of the administration procedure is being followed! complied with. Therefore, the server checks/ determines if the input information/ treatment parameters from the mobile device is being submitted timely. If an input information is missing or is not submitted within an expected/ predetermined time, an ad hoc instruction and/or warning can be submitted to the mobile device, notifying or warning the user. The warning is prompted/ indicated on the mobile device, in order for the user to record/ input/ submit the missing information/ parameter promptly. That way, a compliance with the protocol of documentation, more specifically the capturing and recording of the correct input information at the right time, can be ensured. Expressed differently, by comparing the time stamps and the status information provided by the at least one infusion pump with the time stamps and input information from the mobile device, i.e. by analyzing the status information, the input information and the respective time stamps, the server can verify compliance with the protocol of documentation of the administration procedure and give out warnings to the user, if necessary.

Advantageously, by using the status information with additional time stamp information from the infusion pump, instructions can be sent to the mobile device ensuring that the input information/ measured parameters are taken and submitted at the correct time/ in the correct order. In other words, a timely coordination of the administration and the recording of parameters can be facilitated. Additionally, by receiving the information from the mobile device with time stamp information, the timely recording of parameters can be ensured.

In another further preferred embodiment of the disclosure, the drug administration information can comprise identification information and the server can be configured to automatically link the drug administration information to the protocol of documentation of the administration procedure via the identification information. This facilitates a necessary assigning/ linking process between the drug administration information, preferably received from the hospital information system, and the administration procedure protocol. Alternatively, said assigning process can also be performed manually by the user.

In another further preferred embodiment of the disclosure, the input information received by the mobile device can be binary and/or descriptive comprising numeric parameters/ values or text data. Instructions or queries that can be answered with a simple yes or no can be displayed on the mobile device, confronting the user, thus, requiring binary input information from the user.

In another further preferred embodiment of the disclosure, the status information provided by the at least one infusion pump can comprise data about an infusion start time and/or an infusion end time and/or an (infusion) flow rate and/or an administered drug dose and/or a drug name and/or an infused volume and/or an administration time. These parameters may be consulted/ provided in real time.

Preferably, the server can be configured to determine if the input information provided by the mobile device is plausible and submits the warning to the mobile device if the input information is not determined to be plausible by the server. This provides an additional safety layer for the medical system, since the user is warned/ notified about erroneous/ illogical input information that would falsify the administration procedure, in particular an entire clinical trial process.

Further preferably, in a case that an intravenous medication is being administered by the at least one infusion pump, the disclosed medical system can be capable of sending a programming proposal/ order to the infusion pump, in particular via a Wi-Fi network, based on the drug administration information, in terms of drug name, infusion rate, and volume to be infused, and can also allow detailed traceability of the infusion process, containing information about changes in the infusion rate, actual infusion time, and administration volume. In yet another preferred aspect of the disclosure, the medical system is configured to automatically generate a detailed traceability treatment report as a summary of the administration procedure, once the administration is finished, that can be used as an outline of the treatment for the promoter. Preferably, said treatment report comprises patient data, the drug administration information, the infusion pump status information, including time stamps, as well as the treatment parameters registered as input information, including time stamps.

In a further advantageous aspect of the disclosure, treatment parameters may be recorded and submitted automatically from the mobile device to the server. Alternatively or additionally, measurements representing the input information may be taken automatically and submitted to the mobile device and server automatically, meaning a manual measurement by the user is not necessary, thus, enabling a real time communication with the at least one infusion pump and the mobile device, by the server.

The disclosure further relates to a method of documenting and managing a drug administration procedure, comprising the following steps: a) operating at least one infusion pump to administer an infusion to a patient/ administering an infusion to a patient by at least one infusion pump, b) providing status information of the at least one infusion pump to a server, c) receiving input information from a user by a mobile device, d) providing the input information to the server by the mobile device, e) determining whether a protocol of documentation of the administration procedure is followed by the server, and f) submitting a notification comprising an instruction and/or a warning/ notice to the mobile device, wherein steps e) and f) are preferably performed based on the status information and on the input information.

In an advantageous aspect of the disclosed method, it can further comprise a system configuration process that precedes the administrating method step a). This configuration process may comprise the steps: creating an administration protocol comprising instructions about when and how treatment parameters should be registered by a mobile device based on drug administration information, e.g. from a hospital information system; and saving the administration protocol to the server; and preferably an additional approval step in which the administration protocol is approved by an additional user (approver) profile before the drug administration steps are initiated.

Preferably, the method further comprises the steps: analyzing/ comparing the status information provided by the at least one infusion pump and/ with the input information received by the mobile device to determine if the protocol of documentation of the administration procedure is being followed! complied with, by the server, and/or determining if the input information received by the mobile device is submitted in an established order and/or in a timely manner, by the server.

The method can also comprise a final step: creating a (traceability) treatment report of the administration procedure, comprising patient information, the drug administration information, the input information, the status information of the infusion pumps, as well as the time stamp information regarding the input information and the status information.

It is to be understood that all the above-mentioned configurations, functions and suitabilities of the medical system according to the present disclosure may be reformulated in method steps and, preferably, are intended to be part of the method according to the present disclosure. Therefore, the disclosed method may be a method performed with the above-described medical system.

The disclosure further relates to a computer program comprising instructions which, when executed by a computer, cause the computer to perform the method as described above, in particular the following method steps: a) operating at least one infusion pump to administer an infusion to a patient/ administrating an infusion to a patient by at least one infusion pump, b) providing status information to a server by the at least one infusion pump c) receiving input information from a user by a mobile device d) providing the input information to the server by the mobile device e) determining whether a protocol of documentation of the administration procedure is followed, by the server, and f) submitting a notification comprising an instruction and/or a warning/ notice to the mobile device, by the server, wherein steps e) and f) are preferably performed based on the status information and on the input information.

### Brief description of the drawings

In the following, the disclosure will be described in greater detail by means of the accompanying drawings.
Fig. 1 is a schematic view of a medical system with a server, a hospital information system, at least one infusion pump and a mobile device, according to a preferred embodiment of the present disclosure; and
Fig. 2 is a flow diagram showing the method of documenting and managing a drug administration procedure according to the preferred embodiment of the present disclosure.

It should be noted that the figures are of a schematic nature only and are intended solely for the purpose of understanding the present disclosure. Furthermore, the features of the individual embodiments may be interchanged and may occur in any particular combination.

### Detailed description of the drawings

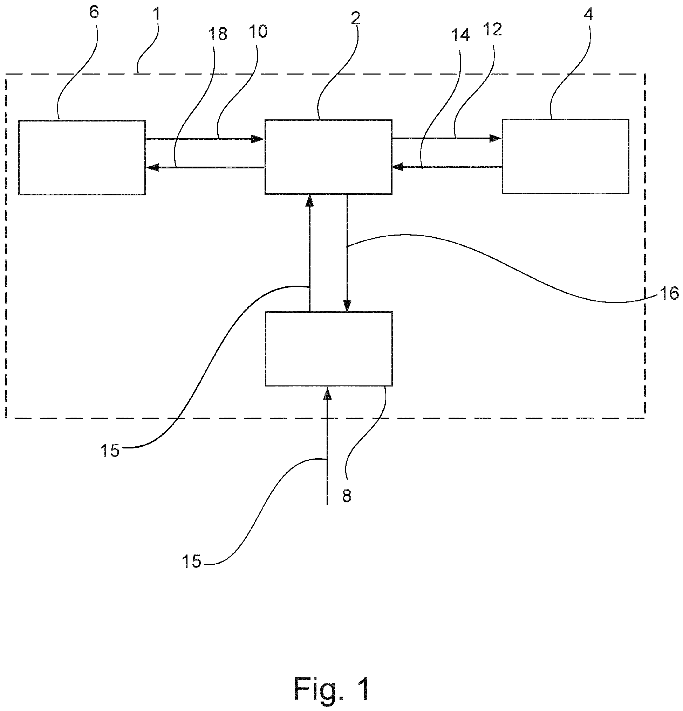

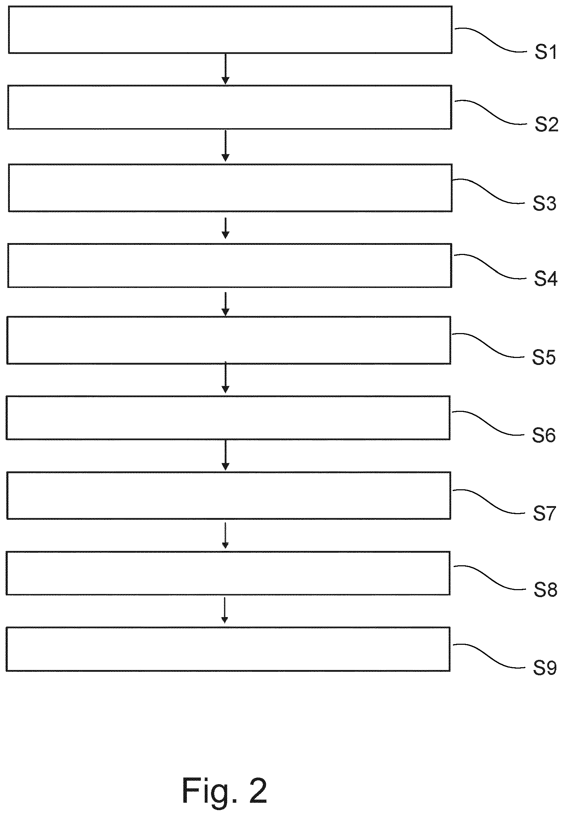

In the following, the present disclosure is described with reference to the preferred embodiment with reference to Figures 1 and 2. Figure 1 shows a medical system 1 in a schematic view and Figure 2 shows a flow diagram of an exemplary documentation and managing process sequence for a drug administration procedure, in particular a clinical trial.

Figure 1 shows a preferred embodiment of a medical system 1 with a server 2, at least one (medical) infusion pump 4 (there may be provided a plurality of infusion pumps 4), a hospital information system 6, and a mobile device 8, in a schematic view. The server 2 is configured to have access to an administration documentation protocol, in particular a clinical trial protocol, that needs to be followed during the administration procedure and is in data communication with the at least one infusion pump 4, the hospital information system 6, and the mobile device 8. It is also possible that a multiple number of mobile devices 8 are in data communication with the server 2. The server 2 is configured to receive drug administration information 10 that comprises, for instance, drug name, recommended dosage rate of the set of drugs to be administered to the patient following a specific order of administration/ a detailed systematic plan. Preferably, the drug administration information 10 can be stored on the server 2 for future administration procedures. The server 2 is configured to send infusion order information 12 based on the drug administration information 10 and the administration protocol to the at least one infusion pump 4, allowing the infusion pump 4 to initiate a drug administration process to a patient. The at least one infusion pump 4 sends infusion status information 14 to the server 2, for example, information about the infusion rate, actual administration volume of the drug, and the administered dose of the drug. The infusion status information 14 can also comprise comparisons between these real-time/ actual values and theoretical values that are based on the drug administration information 10. Additionally, the infusion status information 14 comprises time stamp information/ values and the server 2 is configured to receive the time stamp information. The time stamps comprise information about at what exact time the respective values of/for the infusion status information 14 have been taken/ measured (by the infusion pump(s) 4).

The mobile device 8 is configured to receive input information 15 throughout the administration procedure, from a user that is then submitted to the server 2 by the mobile device 8. Said input information 15 comprises data about whether individual treatment parameters have been registered/ submitted or not (by the user), and/or values of the treatment parameters that are, particularly measured/noted by the user, and entered into the mobile device 8. Examples for treatment parameters that can be registered by the mobile device 8 are electrocardiograms, temperature, or vital parameters. In this preferred embodiment of the disclosure, the input information 15 also comprises time stamp information, in other words additional data about when the input information 15 has been input by the user. The server 2 receives the input information 15 and is configured to send a notification 16 to the mobile device 8, based on the input information 15 received, the status information 14 from the infusion pump(s) 4 as well as the administration protocol. The notification 16 can comprise an instruction and/or a warning for the user. Therefore, the server 2 is configured to analyze and compare the (infusion) status information 14 of the infusion pump(s) 4, the administration protocol that is partially based on the drug administration information 10, and the input information 15 submitted by the mobile device 8, with each other, and determine if the administration protocol is being complied with, preferably in real-time. Based on this result, the server 2 is configured to provide the notification 16 in the form of instructions/ warnings to the user via the mobile device 8. In this preferred embodiment, the server 2 is also configured to provide administration recording information 18 of the currently active or finished administration procedure to the hospital information system 6 for future use.

Figure 2 is a flow diagram showing the method of documenting and managing a drug administration procedure according to the preferred embodiment of the present disclosure. In a first step S1, at least one infusion pump 4 is operated to administer a drug infusion to a patient/ a drug infusion is administered to a patient by the at least one infusion pump 4. In particular, the drug infusion is based on the drug administration information 10 by the hospital information system 6. In a second step S2, the at least one infusion pump 4 provides status information 14 to the server 2, preferably including time stamp information. In a third step S3, the input information 15, preferably comprising time stamp information, is received by the mobile device 8 from a user and the mobile device 8 provides the input information 15 to the server 2 in a fourth step S4. The server 2 then analyzes and compares the status information 14 provided by the at least one infusion pump 4 and/ with the input information 15 received by the mobile device 8 to determine if the protocol of documentation of the administration procedure is being followed, in a fifth step S5. In a sixth step S6, the server 2 determines if the input information 15 received by the mobile device 8 is submitted in an established order and/or in a timely manner. In a seventh step S7, the server 2 determines if the input information 15 provided by the mobile device 8 is plausible, preferably based on past records stored on the server 2 or in the hospital information system 6. The order of the steps S5, S6, and S7 is interchangeable. In the following step S8, the server 2 submits a notification 16 comprising instructions and/or warnings to the mobile device 8, depending on the results of steps S5, S6, and/or S7. Lastly, in step S9, the server 2 (automatically) generates a detailed traceability treatment report with the different information, including time stamps, of the just completed administration procedure. The report preferably also includes patient data, like the patient name and the age of the patient, as well as information about who registered the input information via the mobile device 8. Depending on the treatment parameters, other information may also be displayed, such as an identification, vital signs, etc.

A more specific example of the documentation and managing method according to the preferred embodiment of the disclosure is described herein below. In step S1 of this example, the at least one infusion pump 4 is operated to begin to administer paracetamol to the patient at a specific time (start time of the administration). The infusion rate and the administered dose, e.g. 1000 ml, as well as the infusion duration are based on the drug administration information 10 provided by the hospital information system 6. In the second step S2, the at least one infusion pump 4 provides status information 14 to the server 2, for example the real/ currently administered volume of paracetamol. The status information 14 of step S2 includes time stamp information, that means the exact time when the currently administered volume of paracetamol has been measured and submitted by the at least one infusion pump 4. In the third step S3, the user is instructed (by the mobile device 8) to provide input information 15 via the mobile device 8 and the input information 15 that is received by the mobile device 8 from the user, e.g. a body temperature of the patient or other vital sign information, that also includes time stamp information (at what exact time the mobile device 8 received the input information 15), is provided to the server 2 in a fourth step S4. The server 2 then analyzes and compares the currently administered volume of paracetamol provided by the at least one infusion pump 4 and/ with the patient vital sign information submitted by the user and determines if the protocol of documentation of the administration procedure is being complied with, in the fifth step S5. In the following sixth step S6, the server 2 determines if the vital sign information received by the mobile device 8 is submitted in an established order and/or in a timely manner by the user, for example if the user has measured and input the necessary parameter information at a predefined time/ order according to the administration protocol. In step S7, the server 2 determines if the currently administered volume of paracetamol is plausible. If any issues or flaws are recognized by the server 2 during step S5 and/or step S6 and/or step S7, the server 2 then sends a notification 16 comprising instructions and/or warnings to the mobile device 8, and therefore to the user, in the step S8, in order to counteract said flaws, based on the status information 14, e.g. the currently administered volume of paracetamol, and on the input information 15, e.g. the vital sign information submitted by the user, both also including time stamp information.

After the administration of paracetamol, an administration of the next drug, e.g. Dexchlorpheniramine with an exemplary flow rate of 102 ml/h can be started during the same administration process. The flow rate and administration dose of Dexchlorpheniramine are also based on the drug administration information 10 provided by the hospital information system 6 and steps S1 to S8 are repeated.

The method is stored on a computer program within a controller of the server 2.

### List of reference signs

- 1: medical system
- 2: server
- 4: infusion pump
- 6: hospital information system
- 8: mobile device
- 10: drug administration information
- 12: infusion order information
- 14: infusion status information
- 15: input information
- 16: notification
- 18: administration recording information

- S1: step operating at least one infusion pump
- S2: step providing status information
- S3: step receiving input information by the user
- S4: step providing input information to the server
- S5: step comparing status information and input information
- S6: step determining if the input information is provided in an established order
- S7: step determining if the input information is plausible
- S8: step submitting a notification to the mobile device
- S9: step generating a treatment report

## Claims

1. A medical system (1) for documenting and managing an administration procedure of drugs, comprising:
a server (2);
at least one infusion pump (4) configured to administer an infusion to a patient and comprising status information (14), the at least one infusion pump (4) being in data communication with the server (2) and providing the status information (14) to the server (2); and
a mobile device (8) configured to receive input information (15) from a user, the mobile device (8) being in data communication with the server (2) and providing the input information (15) to the server (2); wherein
the server (2) is configured to determine whether a protocol of documentation of the administration procedure is followed and to submit a notification (16) comprising an instruction and/or a warning to the mobile device (8) based on the status information (14) and on the input information (15).

2. The medical system (1) according to claim 1, wherein the server (2) is configured to store drug administration information (10) and/or to receive the drug administration information (10) from a hospital information system (6) that is in data communication with the server (2).

3. The medical system (1) according to claim 2, wherein the server (2) is configured to send infusion order information (12) based on the drug administration information (10) to the at least one infusion pump (4).

4. The medical system (1) according to claim 3, wherein the server (2) is configured to receive an approval input that approves the protocol of documentation of the administration procedure and/or any changes made to the protocol of documentation before sending the infusion order information (12) based on the drug administration information (10) to the at least one infusion pump (4).

5. The medical system (1) according to any one of claims 2 to 4, wherein the drug administration information (10) comprises identification information and the server (2) is configured to automatically link the drug administration information (10) to the protocol of documentation of the administration procedure through the identification information.

6. The medical system (1) according to any one of claims 1 to 5, wherein the status information (14) provided by the at least one infusion pump (4) and/ or the input information (15) provided by the mobile device (8) comprise time stamp information.

7. The medical system (1) according to claim 6, wherein the server (2) is configured to determine if the input information (15) is being received in an established order and/or in a timely manner by the mobile device (8), based on the time stamp information, and to submit a warning to the mobile device (8) if the input information (15) has not been received in the established order or in a timely manner.

8. The medical system (1) according to any one of claims 1 to 7, wherein the server (2) is configured to analyze/ compare the status information (14) provided by the at least one infusion pump (4) and/ with the input information (15) received by the mobile device (8) to determine if the protocol of documentation of the administration procedure is being followed.

9. The medical system (1) according to any one of claims 1 to 8, wherein the input information (15) received by the mobile device (8) is binary and/or descriptive comprising parameter values or text data.

10. The medical system (1) according to any one of claims 1 to 9, wherein the status information (14) provided by the at least one infusion pump (4) comprises infusion start time and/or infusion end time and/or infusion flow rate and/or administered drug dose and/or drug name.

11. The medical system (1) according to any one of claims 1 to 10, wherein the server (2) is configured to determine if the input information (15) provided by the mobile device (8) is plausible and submits the warning to the mobile device (8) if the input information (15) is not determined to be plausible by the server (2).

12. The medical system (1) according to any one of claims 1 to 11, configured for documenting and managing a clinical trial.

13. A method of documenting and managing a drug administration procedure, the method comprising the steps:
Operating (S1) at least one infusion pump (4) to administer an infusion to a patient;
Providing (S2) status information (14) to a server (2) by the at least one infusion pump (4);
Receiving (S3) input information (15) from a user by a mobile device (8);
Providing (S4) the input information (15) to the server (2) by the mobile device (8);
Determining whether a protocol of documentation of the administration procedure is followed by the server (2); and
Submitting (S8) a notification (16) comprising an instruction and/or a warning to the mobile device (8).

14. The method according to claim 13, further comprising the steps Analyzing (S5) the status information (14) provided by the at least one infusion pump (4) and the input information (15) received by the mobile device (8) to determine if the protocol of documentation of the administration procedure is being followed, by the server (2); and/or
Determining (S6) if the input information (15) received by the mobile device (8) is submitted in an established order and/or in a timely manner, by the server (2).

15. A computer program comprising instructions which, when executed by a computer, cause the computer to perform the following method steps, in particular according to the method according to claim 13 or 14:
Operating (S1) at least one infusion pump (4) to administer an infusion to a patient;
Providing (S2) status information (14) to a server (2) by the at least one infusion pump (4);
Receiving (S3) input information (15) from a user by a mobile device (8);
Providing (S4) the input information (15) to the server (2) by the mobile device (8);
Determining whether a protocol of documentation of the administration procedure is followed, by the server (2); and
Submitting (S8) a notification comprising an instruction and/or a warning to the mobile device (8), by the server (2).
